**Europäisches Patentamt**

**European Patent Office**

**Office européen des brevets**

(19)

(11) Publication number : **0 448 438 A2**

(12) # EUROPEAN PATENT APPLICATION

(21) Application number : **91400616.8**

(22) Date of filing : **06.03.91**

(51) Int. Cl.$^5$ : **A61B 5/113**

(30) Priority : **07.03.90 IL 93675**

(43) Date of publication of application :
**25.09.91 Bulletin 91/39**

(84) Designated Contracting States :
**BE DE FR GB IT**

(71) Applicant : **THE SLEEP DISORDERS
DIAGNOSTIC AND TREATMENT CENTER LTD.
Gutwirth Bld., Technion City
Haifa 32000 (IL)**

(72) Inventor : **Zomer, Jacob
5, Hachita St.
Rehovoth (IL)**

(74) Representative : **Levesque, Denys et al
Cabinet Beau de Loménie 55, rue
d'Amsterdam
F-75008 Paris (FR)**

(54) **Device for sensing respiration movements.**

(57)    A device for indicating the respiration movements on a recorder, without being affected by external pressure, comprises at least two piezoelectric transducers (7, 7') which are built oppositely each against the other, embraced by a metallic member, and a membrane (8) possessing a wave-like shape. The entire device is enclosed in a housing (2) open at its two ends. Upon stretching said membrane (8), the piezoelectric transducers (7, 7') generate an electric signal, according to the respiration movements, any external pressure being cancelled by the action of the transducers. The electric signal is indicated on a strip chart recorder. The device has the advantages of prolonged use time and very high sensitivity which imparts high accuracy and therefore provides correct respiration data.

# FIG.3

EP 0 448 438 A2

The present invention relates to an improved device for indicating the respiration movements. More particularly, the invention relates to an improved device which provides a non-quantitative but accurate indication of the respiration movements of a person without requiring a direct skin contact thereon.

## BACKGROUND OF THE INVENTION

The problem of getting an indication on the respiration movements of a person is quite important. Frequently, it is required to monitor a patient breathing and to actuate an alarm means when the respiration ceases.

There are various devices for providing this indication. A well-known device comprises a rubber tube which is filled with mercury, or a saline solution, and closed at the two ends. The tube is fitted around the chest of a patient, the indication of respiration movements being obtained by determining the voltage generated accross the tube when a fixed current is passed through it. The content of the tube acts as a variable resistor, the resistance of which changes as the tube is stretched or contracted upon breathing. A similar device was later on developed in which said tube contains a solid substance such as graphite powder instead of a liquid. The main disadvantage of these devices is the fact that the resulted generated voltage is incorrect since it includes also the pressure of the body on the tube. Furthermore, since the elastic tube is not so robust, sometimes it can be ruptured, the liquid or powder going out therefrom and thus providing- inaccurate results from the signal obtained.

Another group of devices for the respiration movements indication, is based on two small electrodes which can be taped on the chest of the patient in contact with the skin while an electric current is passed between them. A varying voltage is detected, which varies with the breathing movements due to the varying resistance of the chest wall itself.Among the main disadvantages of these devices it should be mentioned the requirement of a direct contact with the skin, i.e. a prior removal of the clothes, which in emergency or casualty situation is actually impossible and of course there is a danger of electrocution.

A more interesting device was later on developed which does not require a direct contact with the skin and can be attached outside the clothing. According to this device, as described in the U.K. Patent No. 2,181,555, a piezoelectric transducer is mounted on a flexible support in such a manner that it can be bent and will generate an electrical signal in response to the displacement of a patient's chest during breathing. The main disadvantage of this device is that during its stretching, the flexible support with the piezoelectric transducer become concave to fit the shape of the body so that the electrical signal which is recorded is not a true representation of the stretching forces but is rather a result of the bending forces due to respiration movements. Furthermore, the results will be completely inaccurate in case that an external perpendicular stress not connected with the respiration movements will be present. This might occur, for example, while a person is moving during sleeping on a solid member, such as his own hand.

A quite similar device is described in the PCT Patent Application AU 85/00163. According to this device, it is claimed a sensor provided with a flexible member and an elongated rigid member disposed thereon. A piezoelectric transducer, is disposed with one end of the rigid member. The disadvantage of this device is, again, the fact that the sensor will provide also other external stresses on the sensor which are not connected with the respiration movements.

The above brief review shows the long felt need for an improved device which will provide an accurate indication of the respiration movements and will not be influenced by external factors.

It is an object of the present invention to provide a simple device for obtaining a true indication of the breathing movements.It is a further object of the present invention to provide a simple device which indicates only the breathing movements and does not require its direct contact with the skin. It is yet another object of the present invention to provide a simple device which is capable of sensing accurately the respiration movements with the influence of artifacts due to bending or pressing forces other than the stretching forces that are applied by the respiration movements.

## BRIEF DESCRIPTION OF THE INVENTION

The invention consists of a device for indicating the respiration movements on a recorder, without being affected by external pressure, which comprises at least two piezoelectric transducers mounted on a metallic member enclosed in a housing, each pair of said transducers being built oppositely each against the other, and a membrane possessing a wave-like shape which its tangency creates angles of at least 5° in alternating directions, said membrane being stretched at the two ends to a flexible band for providing an elastic belt-like member surrounding a patient's clothing, the external pressure being cancelled by the action of the differentially transducers, the device generating an electric signal, according to the respiration movements, said signal being indicated on a recorder. The recorder may be any strip chart recorder possessing a high input resistance such as a common electroencephalograph or any other known instrument as used in sport activities.

According to one embodiment, the membrane is located between the two piezoelectric transducers. In this manner, the alternating angles transform the external stretching resulting from the respiration movements into pressing and bending the transducers.

According to another embodiment, the membrane is located on the external side of one of said piezoelectric transducers, in which case said angles are located on the external side of the membrane, causing bending the transducer upon streching the belt.

The elastic belt-like member is provided with attachment means at its two ends by adhesive tapes or any other fastener means.

According to a most preferred embodiment, the entire device is located in a housing, open at its two ends, the entire space being filled with an elastic constituent such as glue, any type of silicon rubber, etc.

The electrical output from the two piezoelectric transducers is fed to a low pass filter from which it is transferred to a voltage divider producing a desired output signal range. This signal is supplied to a cable leading to a pair of plug-type connectors linked with a recorder.

## BRIEF DESCRIPTION OF THE DRAWINGS

The invention will be more fully understood, and other features and advantages thereof will become apparent from the description given below of an exemplary embodiment of the invention, in which:

**Figure 1,** is a cross-section of the device wherein the membrane is located between the two piezoelectric transducers, enclosed in a housing.

**Figure 2,** is a cross section of the device wherein the membrane is **located** outside one of the piezoelectric transducers, enclosed in a housing.

**Figure 3,** is a view of the internal construction of the device which appears in Figure 1.

**Figure 4,** shows 3 graphs which illustrate the curves of respiration signal recorded by a recorder connected to the output of the signal conditioning circuit, wherein only graph C is that of the device according to the present invention, the other two graphs being presented only for comparison purposes.

One of the advantages of the device according to the present invention is the presence of two piezoelectiic transducers and said membrane with a wave-like shape which its tangency creates angles of at least 5° and preferably above 20°, which cause that only the respiration movements on the length axis would impart the electric signal, the differential construction suppressing the stresses provided from any other source. It was found that the minimum angle of 5° for the tangency of the wave like shape in the said membrane is quite critical in order to achieve the differential effect according to the present invention which will be very sensible to stretches but not sensible to perpendicular stresses or accelerations due to the body movements. Another advantage of the present device is its rigid shape which imparts a prolonged use time.

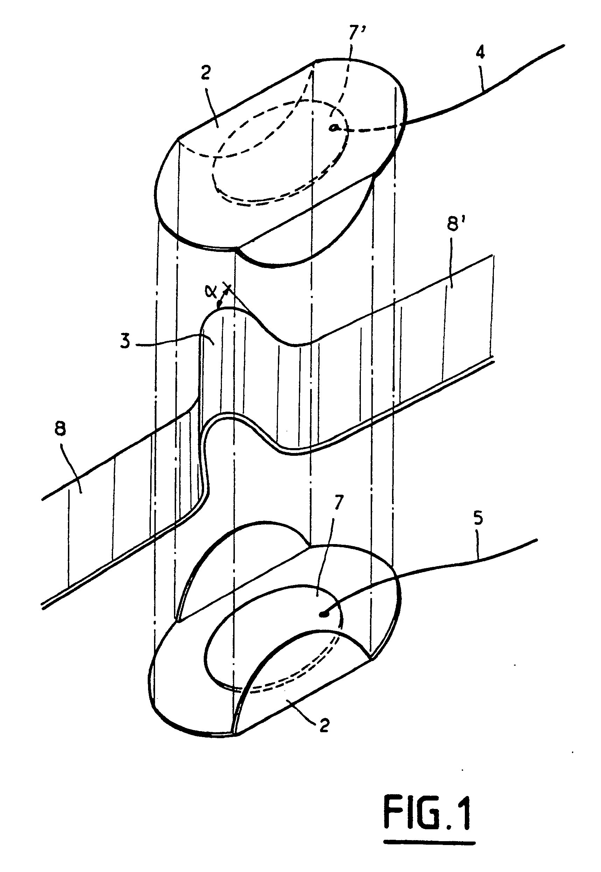

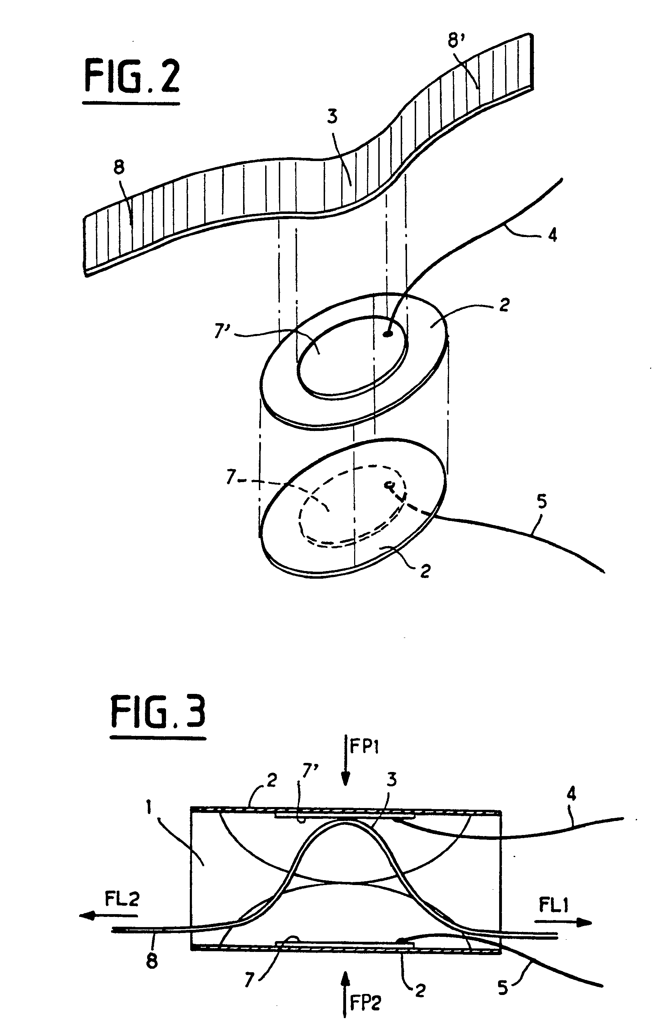

Referring now in more detail to the attached drawings, in Figure 1 it can be noticed the two piezoelectric transducers (7 and 7'), welded oppositely on a metal sheet, separated by a membrane (3) possessing a wave-like shape with a series of angles ($\alpha$) of above 20°. The two ends of the membrane (8 and 8') are streched so that the angles are substantially eliminated thus forming a belt-like which is elastic to bend but not to stretches. Typical examples of materials useful for the belt are selected from known common plastic materials which are elastic such as PVC, polyethylene, polyamide, etc. The device is enclosed in a housing (2) open at its two ends, the entire space being filled with a common resilient constituent such as glue or silicon rubber. Two electrical leads (4 and 5) extend from the side surface of the metallic member (Figure 3) embracing the piezoelectric transducers. In Figure 2, said membrane is outside one of the piezoelectric transducers.

In either one embodiment, the transducers are driven by the membrane through said resilient filling medium.

In Figure 3 it can be noticed the two undesired perpendicular forces FP1 and FP2 which are equal, causing that same stresses will result and therefore are eliminated due to the differential addition. These two forces are acting onto the two piezoelectric transducers (7 and 7') separated by the membrane (3) with the wave-like shape. The two electrical leads (4 and 5) are also shown. The two longitudinal forces FL1 and FL2 are applied onto the belt according to the expansion of the chest during the respiration movements. As a result of these forces, the two piezoelectric transducers will act in the same direction and thus will impart the resulted voltage to the electrical leads connected to the metallic member.

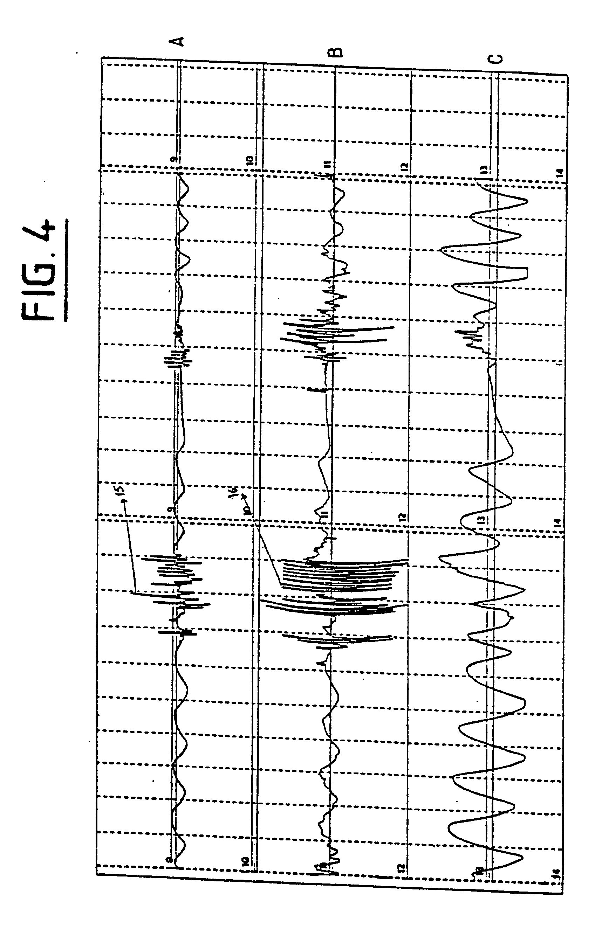

In Figure 4 threre are presented three graphs which show the curves of respiration movements of a same person, as obtained on the same recorder, wherein:

**Graph A** illustrates the curve obtained with a device comprising two piezoelectric transducers without a membrane (for comparison purpose).

**Graph B** illustrates the curve obtained with a device comprising only one piezoelectric transducer with a membrane possessing the specific wave-like shape (for comparison purpose).

**Graph C** illustrates the curve obtained with a device according to the present invention.

In the Graphs A and B the artifacts regions (15 and 16) clearly appear. In Graph A, the device used contained two piezoelectric transducers but without the membrane, while in Graph B the device used contained the membrane (as used in the present invention) but only with one single piezoelectric transducer. As appears in a clear manner from the three graphs, it is only with the device according to the present invention, as shown in Graph C, that the artifact regions do not exist due to the membrane with its wave-like shape. It is only in this case that the perpendicular forces are eliminated and in this manner the respiration movements alone will provide a true representation.

Among the advantages of the device according to the present invention, in addition to the true representation of the respiration movements, it should be mentioned the fact that it is not necessary to remove the patient's clothes and only a firm contact between the device and the clothes on the chest area will be sufficient to obtain an accurate graph representation. This firm contact may be achieved by connecting the ends of the two bands by adhesive tapes or any other fastener means.

Another advantage of the device results from its rigidity construction due to its mode of operation, which does not require its bending even when the belt is applied onto a large circumference. In this manner it can be easily installed in a protective rigid housing. As will be realized, any bend of the belt will impart significant inaccuracies in the signal produced on the recorder.

The device for indicating the respiration movements according to the present invention, does not incur any encumbrance to the person on which it is applied, has extremely high sensitivity and yet is of simple and inexpensive construction. It is self-balancing and particularly easy to install and operate to provide highly accurate and useful respiration data.

While the invention has been described in connection with certain preferred embodiments, it will be understood that it is not intended to limit the invention to those particular embodiments. On the contrary it is intended to cover all alternatives, modifications and equivalents as may be included in the appended Claims. Some specific components figures and types of materials are mentioned, but is is to be understood that such component values, dimensions and types of materials are, however, given as examples only and are not intended to limit the scope of this invention in any manner.

**Claims**

1. A device for indicating the respiration movements on a recorder, without being affected by external pressure, which comprises at least two piezoelectric transducers mounted on a metallic member enclosed in a housing, open at its two ends, each pair of said transducers being built oppositely each against the other and a membrane possessing a wave-like shape which its tangency creates angles of at least 5° in alternating directions, said membrane being streched at the two ends to a flexible band for providing an elastic belt-like member surrounding a patient's clothing, the external pressure being cancelled by the action of the differentially transducers the device generating an electric signal according to the respiration movements, said signal being indicated on a recorder.

2. The device according to Claim 1, wherein said membrane is located between the two piezoelectric transducers.

3. The device according to Claim 1, wherein said membrane is located on the external side of one of said piezoelectric transducers.

4. The device for indicating the respiration movements according to anyone of claims 1 to 3, wherein said angles in the membrane are above 20°.

5. The device for indicating the respiration movements according to anyone of claims 1 to 4, wherein said housing is filled with an elastic material.

6. The device for indicating the respiration movements according to claim 5, wherein said elastic material is selected from glue and silicon rubber.

FIG.1

# FIG.2

# FIG.3

# FIG. 4

EP 0 448 438 A2